# EUROPEAN PATENT SPECIFICATION

(11) **EP 1 274 674 B1**
(45) Date of publication and mention of the grant of the patent: **08.03.2006**
(21) Application number: 01925883.9
(22) Date of filing: 18.04.2001
(51) Int. Cl.: C07C 219/06, A61K 31/21, A61P 25/28, C07C 53/128

(54) **CHOLINE DERIVATIVES FOR THE TREATMENT OF ALZHEIMER'S DISEASE**
CHOLINDERIVATE ZUR BEHANDLUNG DER ALZHEIMERKRANKHEIT
DERIVES DE LA CHOLINE DANS LE TRAITEMENT DE LA MALADIE D'ALZHEIMER

(30) Priority: 21.04.2000 IT MI000898
(43) Date of publication of application: 15.01.2003
(73) Proprietor: UNIVERSITA' DEGLI STUDI DI ROMA "LA SAPIENZA", 00185 Roma (IT); Universita' Degli Studi Di Catanzaro "Magna Graecia", 88100 Catanzaro (IT); Universita' Degli Studi Di Camerino, 62032 Camerino (IT)
(72) Inventor: CARELLI, Vincenzo, Univ. Studi di Roma La Sapienza, 00185 Roma (IT); LIBERATORE, Felice, Univ. Studi Roma La Sapienza, 00185 Roma (IT); SCIPIONE, Luigi, Univ. Studi di Roma La Sapienza, 00185 Roma (IT); CARDELLINI, Mario, Univ. degli Studi di Camerino, I-62032 Camerino (IT); ROTIROTI, D., Univ. Studi Cantazaro Magna Graecia, 88021 Catanzaro (IT); RISPOLI, V., Univ. Studi di Catanzaro Magna Gracia, 88021 Catanzaro (IT)
(74) Representative: Minoja, Fabrizio
(86) International application number: PCT/IT2001/000191
(87) International publication number: WO 2001/081296

(56) References cited:
- WO-A-92/22534
- WILSON, K. JEFF ET AL: "The time dependent UV resonance Raman spectra, conformation, and biological activity of acetylcholine analogs upon binding to acetylcholine binding proteins" J. BIOMOL. STRUCT. DYN. (1991), 9(3), 489-509 , XP001011474
- THANEI-WYSS, PETER ET AL: "Interaction of quaternary ammonium compounds with acetylcholinesterase: characterization of the active site" EUR. J. PHARMACOL., MOL. PHARMACOL. SECT. (1989), 172(2), 165-73 , XP001011377
- HOLATA, J. ET AL: "Chemical transformations of polymers. XVI. Preparation and splitting of poly(methacryloyloxyethyltrimethylammonium hydroxide)" J. POLYM. SCI., POLYM. SYMP. (1974), 47(TRANSFORM. FUNCT. GROUPS POLYM., PRAGUE IUPAC MICROSYMP. MACROMOL., 13TH, 1973), 335-43 , 1974, XP001002927

## Description

The present invention relates to the use of choline derivatives for the treatment of the diseases related with cholinergic central deficit, such as Alzheimer' disease.

More particularly, the invention relates to choline derivatives having pharmacological activity in the remission of the central cholinergic deficit conditions and in the restoration of normal cerebral electric activity.

### INTRODUCTION

Alzheimer's disease (AD) is the most common dementia in the elderly population. It is a neurodegenerative disorder with progressive evolution and it represents 50% of all the irreversible dementia forms. The incidence of the disease increases in subjects above 80 years.

The main symptoms at the onset of the disease are memory disorders with progressive cognitive decline and subsequently behavioral, psychiatric and personality changes.

AD diagnosis is confirmed *post-mortem* in about 80%-90% of cases. *Post-mortem* studies on the brains of AD patients evidenced a substantial degeneration of the subcortex areas, particularly of *nucleus basalis* of Meynert (NBM); degeneration is accompanied by a remarkable reduction of cholinergic neurons present in this area. Brain shows reduced levels of acetylcholine (Ach) and of its specific enzymatic markers, such as *cholineacetyltransferase* (ChAT) and *acetylcholinesterase* (AchE), (Whitehouse et al., 1982; Deeker, 1987).

NBM, together with the other CNS neurotransmission systems, plays an essential role in the cortical activation mechanism, which is the basic electrophysiological mechanism involved in attention and learning. A relationship exists between degeneration of NBM cholinergic neurons and the neurophysiological deficits observed in AD and in normal aging (Whitehouse et al., 1981, 1982; Aremdt et al., 1984; Rinne et al., 1987) as well as the severity of the disorder (Perry et al., 1978).

A number of studies proved that the cholinergic system plays an important role in cerebral cortex activation, which process in CNS is connected with wake and alert behaviours (Lo Conte et al., 1982; Longo, 1996; Miller et al., 1940; Stewart et al., 1984; Moruzzi, 1972). Cholinergic cortical projections of NBM are able to directly modulate activation of neocortex (Lo Conte et al., 1982).

Activation of neocortex is fundamental for normal cognitive processes to occur. This activation is correlated with cortical electroencephalogram desynchronization, i.e. with the presence in the electroencephalogram (EEG) of low amplitude waves, associated with rapid frequency.

Furthermore, activation of the cholinergic system at the basal level is paramount in maintaining a behavioural response of *alert.* NBM stimulation produces, in fact, after a short latency, activation of cortical neurons (Funanhashi, 1983; Stem and Pugh, 1984) and Ach release in neocortex (Casamenti et al., 1986). EEG changes (decrease in frequencies with rapid activity, 12-16 Hz) have been observed in animals subjected to unilateral lesion of NBM.

Clinical studies in elderly subjects as well as in AD patients evidenced a progressive morphological change in EEG, which is characterized by slower cerebral electrical activity; the appearance of slow activity in EEG is due to the prevailing presence of large amplitude waves (EEG synchronisation). Furthermore, a number of studies evidenced the relationship between topography of the characteristic EEG changes and severity of the disease (Soininen et al., 1991; Ono et al., 1992; Kuskowski et al., 1993; Schreiter-Gasser et al., 1993; d'Onofrio et al., 1996; Helkala et al., 1996; Stam et al., 1996; Chiaramonti et al., 1997; Lopez et al., 1997). These EEG changes are associated with the degeneration of basal cholinergic system.

The study of cerebral electrical activity by EEG can be a valuable approach in pharmacological evaluation of new compounds able to counteract AD progress. Qualitative (frequency analysis) and quantitative (spectral analysis) analysis of cerebral electrical activity, carried out by means of computerised, digital EEGraphers, is one the of most advanced tools available at present and offers huge possibilities of clinical application.

Quantitative EEG analysis in AD patients evidenced a remarkable increase in delta and theta activity (waves) and a decrease in alpha and beta rhythms (waves) (Cohen et al., 1985; 1990; Elmstahl et al., 1994).

According to the theory linking Alzheimer's disease to cholinergic dysfunction, many reasearches and clinical studies proved that the administration of acetylcholine precursors can revert mental senile impairment and improve cognitive performances in AD patients (Greenwald et al., 1985; Franco-Maside et al., 1994; de la Morena, 1991; Secades et al., 1995).

The pharmacological intervention aimed at reverting the cholinergic transmission central deficit by use of acetylcholine precursors is at present one of the choice therapeutical approaches. It can improve, at least initially, cognitive and behavioural disorders in AD patients. Such therapeutical approach has lead to the preparation and study of a number of choline derivatives, such as 5'-cytidine-diphosphocholine (CDP-choline) and variously substituted lecithins, whose administration produced rather favorable effects on cognitive functions (Gatti et al., 1992; Ceda et al., 1992; Parnetti et al., 1993; Petkov et al., 1993; Cacabelos et al., 1993).

### Background art

European Journal of Pharmacology (Molecular Pharmacology Section, 172 (1989), 165-173) reports an *in vitro* study of the relation between the structure of 31 quaternary ammonium compounds and their effects on the activity of acetylcholinesterase.
Most of the tested compounds acted as reversible, competitive inhibitors of AchE.

### DISCLOSURE OF THE INVENTION

It has now been found that the compounds of formula (I) 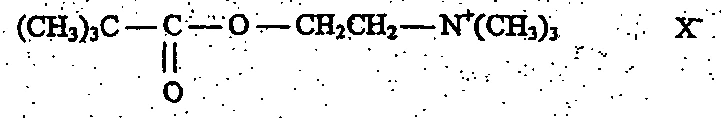
wherein X is iodide or the pivalic acid
can be conveniently used in the treatment of diseases connected with central cholinergic deficit, particularly of Alzheimer's disease or Alzheimer-type diseases, wherein by "Alzheimer's disease" and "Alzheimer-type diseases" any forms of senile or presenile dementia are meant.

It has in fact been found that the compounds of formula (I) are able to restore the normal cerebral electric activity in cholinergic deficit animal models with cholinergic transmission deficiency.

In particular, it has been found that the administration of choline pivalic esters in rats induces EEG desynchronization and quantitative changes in the power and architecture of the EEG tracing, and that said compounds are capable of restoring cerebral electrical activity in NBM lesioned rats.

The activity of the choline derivatives of the invention was studied using CDP-choline as control drug. Choline pivalic esters have shown significantly shorter latency of action as well as longer-lasting effect than CDP-choline. Furthermore, said compounds proved to easily cross the blood-brain barrier and to have no peripheral cholinergic effects. Moreover, admmistration of pivaloylcholine in the presence of *Hemicholinium-3* (Hch3), a potent and selective choline uptake blocker, in the rat, caused no EEG changes. Which are, on the contrary, evoked by administration of the compound alone; even at Hch3 lower dosages, pivaloylcholine caused no changes in the action induced by said choline uptake blocker (behavioural sedation) neither significant changes in BBG. Therefore, one of the action mechanisms of choline derivatives may involve choline specific transport at the cholinergic neuron level. It has also been proved that choline pivalic ester is able to restore the physiological cerebral electrical activity in experimental models of NBM neurochemical lesion, producing EEG desynchronization and quantitative changes in both power and architecture of the EEG tracing, modulating the activity of rapid frequencies and significantly decreasing the amount of low frequencies. The experimental evidence supporting the effects observed with the choline derivatives of the present invention is reported in detail in the Examples.

The obtained data prove that the choline derivatives of the invention have central activity and are useful in the treatment of any pathologies connected with a central cholinergic deficit. More particularly, the obtained data allow to envisage an important role for the tested compounds in the therapy of cognitive impairment and in the improvement of Ap patients life quality.

Therefore, the present invention relates to the use of the compounds of formula (I) in the treatment of central cholinergic deficit diseases, particularly in the treatment of Alzheixner's disease or of Alzheimer-type diseases.

For use in therapy, the compounds of formula (I) will be suitably formulated according to the conventional techniques.

According, to a further aspect, the invention relates to pharmaceutical compositions containing a compound of formula (I) together with pharmaceutically acceptable carriers and excipients. The compositions of the invention can be administered through the oral, parenteral, inhalatory or rectal routes. Suitable administration forms include tablets, capsules, granules, powders, solutions, syrups, solutions for injection, suppositories, transdermal plasters or other controlled-release forms. The pharmaceutical formulations can be prepared with conventional procedures using excipients known in the technique, such as fillers, binders, compression agents, disintegrants, lubricants, stabilizing agents, thickening agents, dispersion agents, dyes, flavours and the like. The dosage of the active ingredient will vary depending on the symptoms and age of the patient, the nature and severity of the disease and the administration route. In principle, effective amount means an amount capable of producing the desired pharmacological effects, i.e. an amount which allows for the active ingredient to cross the blood-brain barrier and to exert its specific activity at the cerebral level, without systemic effects.

In the case of oral administration to an adult patient, the compounds will be generally administered at a daily dose ranging from 1 to 1000 mg, preferably from 5 to 300 mg, in single or multiple dose, for example one to three times a day; in the case of parenteral administration, the dose will range from 0.1 to 500 mg, preferably from 0.5 to 300 mg, one to three times a day.

### DESCRIPTION OF THE FIGURES

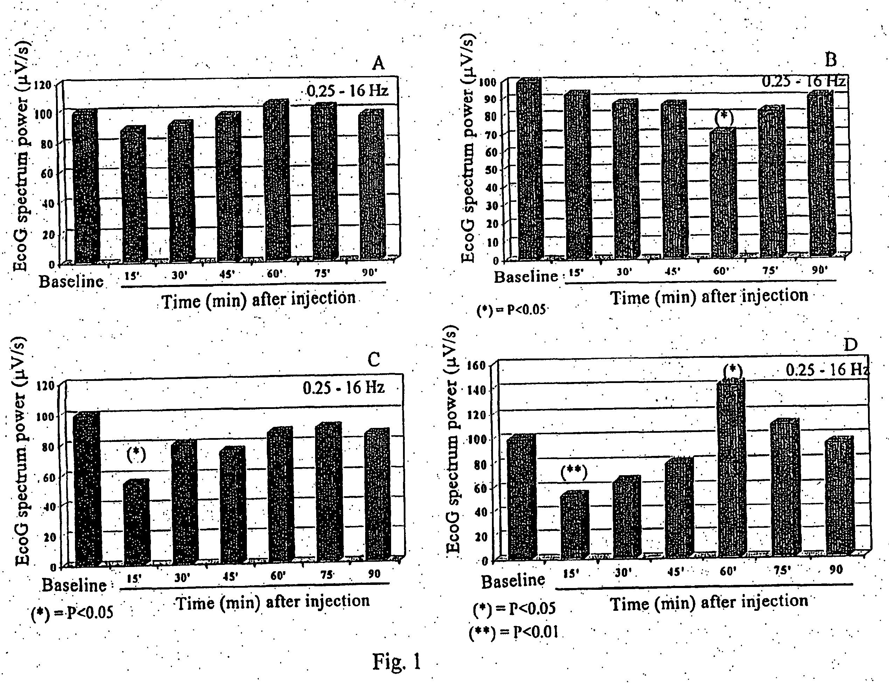

Fig. 1 ―Changes of EEG spectrum power after administration of the choline derivatives. A. Control; B. Effect after administration of CDP-choline (5 mg i.p.); C. Effect after administration of PC-1 (pivaloylcholine iodide); D. Effect after administration of PC-2 (pivaloylcholine trimethylacetate). Time (min) is reported in abscissa, EEG power expressed as µV²/s is reported in ordinate. Graphics report the EEG spectrum total power (0.25-16 Hz).

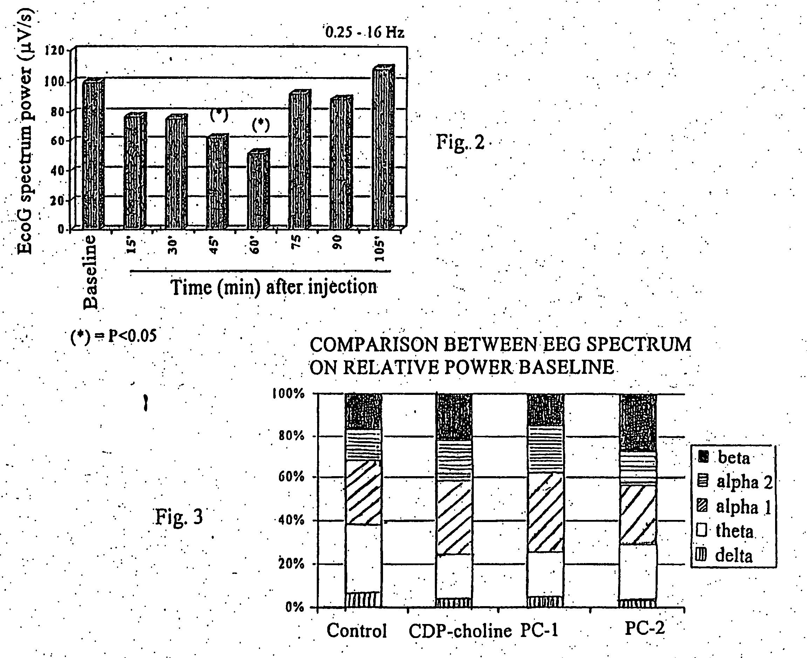

Fig. 2 ― Effect on EEG spectrum power after treatment with atropine (0.1 mg i.p.) administered 15 min before PC-1 (5 mg i.p.). Time (min) is reported in abscissa, EEG spectrum power expressed as µV²/s is reported in ordinate. Graphics report the EEG spectrum total power (0.25-16 Hz).
Fig. 3 - Graphic representation of EEG spectrum in rats. Quantitative analysis of the contribution of each EEG spectrum component to cerebral electrical activity after administration of the choline derivatives. It is noteworthy the decrease in slow components (delta and theta) of the spectrum after administration of PC-1 and PC-2 in relation to EEG desynchronization.
Fig. 4A - Effect on EEG spectrum power after treatment with PC-1 (0.5 mg i.p.).

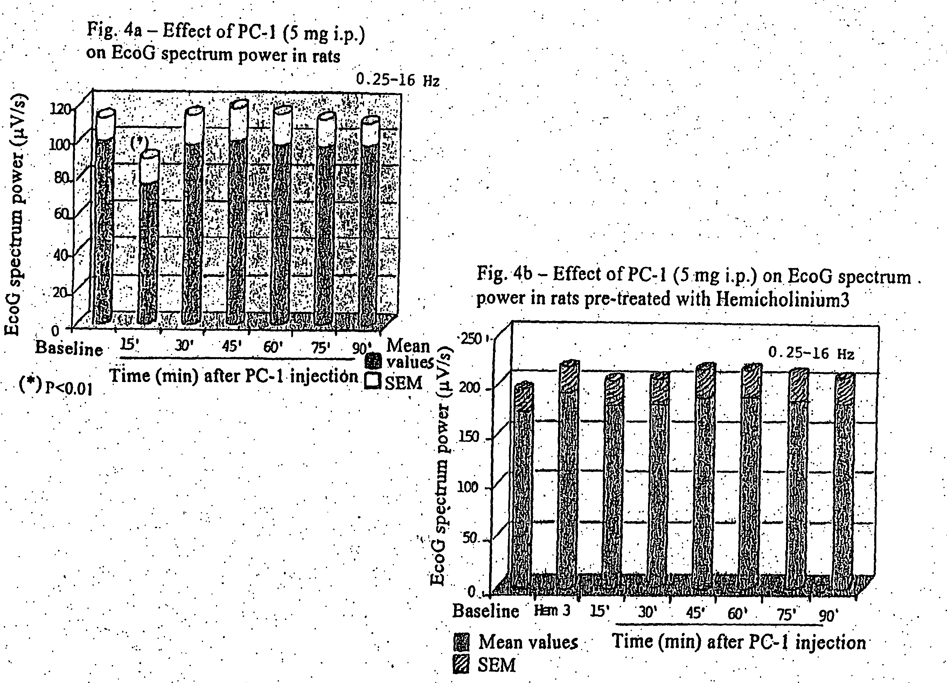

Fig. 4B - Effect on EEG spectrum power after treatment with PC-1 (5 mg i.p.) administered 15 min after microinfusion of Hch3 (0.1 ng/0.5 µl) in the III ventricule. As in Figure 4a, time (min) is reported in abscissa, the EEG spectrum power expressed as µV²/s is reported in ordinate. Graphics report the EEG spectrum total power (0.25-16 Hz).

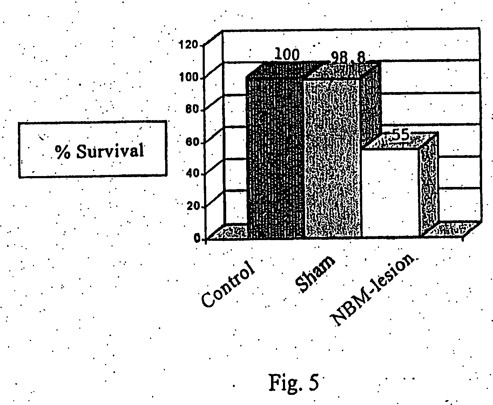

Fig. 5 ― Graphic representation of neuronal loss in NBM after AMPA-induced excitotoxic lesion.

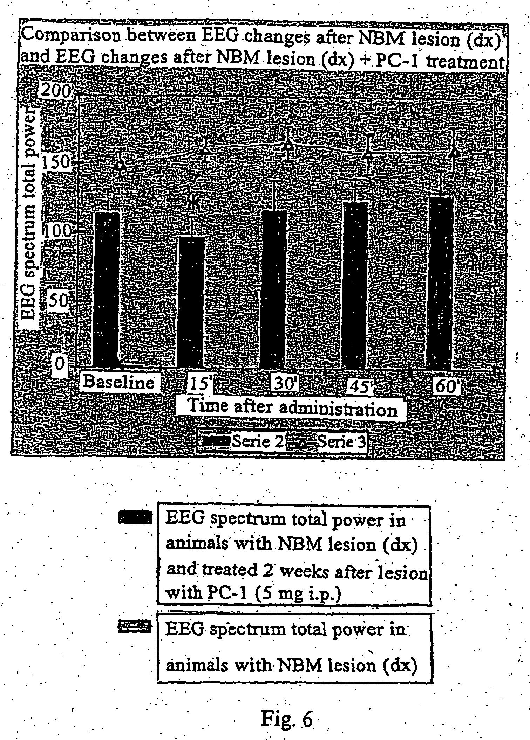

Fig. 6 ― Graphic representation of the effects induced by administration of PC-1 (5 mg i.p.) on EEG spectrum total power in animals subjected to unilateral lesion of NBM (lesion effected by AMPA).

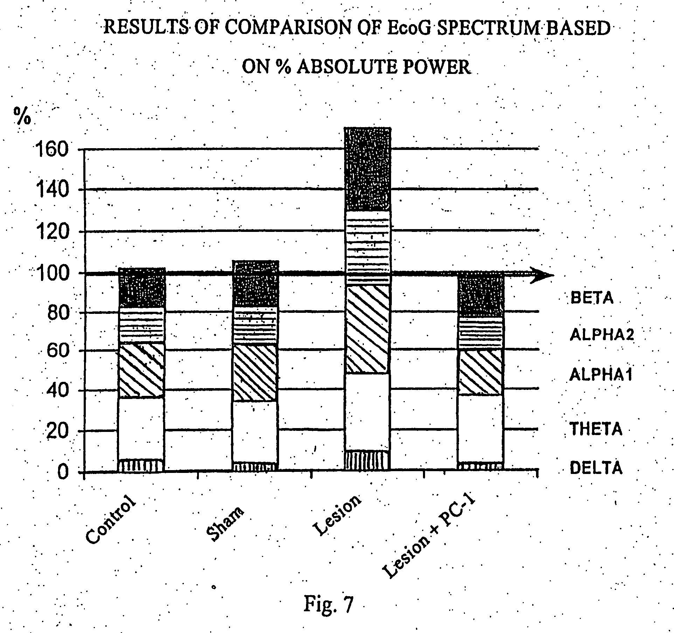

Fig. 7 ― Effect on EEG spectrum total power following NBM excitotoxic lesion (AMPA 1,3 µg/0.5 µl). Comparison between absolute EEG spectrum power in ipsilateral hemispheres and lesion in different groups of rats (control, sham operated, lesioned and lesioned, PC-1-treated). It should be noted that administration of PC-1 (5 mg i.p.) restores both the normal composition of EEG spectrum and the distribution of its frequencies. The following examples illustrate the invention in greater detail.

### EXAMPLES

### Example 1 - Preparation of the compounds of formula (I)

The preparation of pivaloylcholine inorganic salts is described in "Collection Czechoslovak Chem. Commun. Vol. 51, 1986, pp 206-214. Two possible synthesis strategies are reported in the following.

### 1 Pivaloylcholine iodide

A solution of 2.00 g of 2-dimethylamino-ethanol (0.022 mols) and 3.1 ml of triethylamine (0.022 mols) in 20 ml of dry ethyl ether cooled on ice bath, is slowly added with 2.7 ml (0.022 mols) of pivaloyl chloride dissolved in 10 ml of dry ethyl ether. After completion of the addition, the solution is stirred at room temperature for 1 hour, filtered and evaporated; the resulting residue is used directly for the subsequent reaction. Iodomethylation is carried out by diluting the ester with 20 ml of acetone and adding 2 ml of methyl iodide. After stirring for five minutes, a white solid precipitates which crystallizes from ethanol. 2.8 g of a crystalline white product with m.p. 198-200°C are obtained.

### 2) Pivaloylcholine trimethylacetate

Silver trimethylacetate is prepared separately dissolving 3.9 g of sodium trimethylacetate monohydrate (0.031 mols) in 10 ml of ethanol and slowly adding a solution of 5.4 g AgNO₃ (0.031 mols) in 10 ml of an ethanol/water 1:1 mixture. 6 g of a white solid are obtained, which is filtered, washed with ethanol and dried.

3.8 g of pivaloylcholine iodide (0.012 mols) are dissolved in 20 ml of water and added with 2,70 g of silver trimethylacetate (0.012 mols). The precipitated AgI is filtered off and the aqueous phase is evaporated to dryness. The resulting solid residue is hygroscopic and crystallizes from dry EtOH/Et₂O (m.p. 200-202°C).

### Example 2 ― Pharmacological tests

### METHODS

Young male Wistar rats (200-260) were used in the experiments, housed in a standard temperature and humidity conditions, with food and water *ad libitum.*

### 1) Surgical procedures

The animals, anaesthetised with chloral hydrate (400 mg/kg) during the whole experiment, were stereotaxically implanted (Kopft Instruments) with cortical electrodes (n = 4) onto the fronto-parietal region of the neocortex on both cerebral hemispheres, to record EEG. After surgery all animals were housed 48 h to allow a complete recovery.

### 2) EEG spectral qualitative and quantitative analysis

48 Hours after electrode implantation, the animals, placed in a transparent box and freely moving, were connected with an electroencephalograph and the electrocortical activity was continuously monitored and recorded. After a 30 min control time, the compounds under test were intraperitoneally administered and EEG recording was continued for 90- 120 min

EEG was recorded by computerised 24-channels apparatus (Vega 24, Galileo type, ESA-OTE Biomedica, Florence, Italy), while the spectrum activity was quantitatively analysed by a Berg Fourier analyser (ESA-OTE Biomedica, Florence, Italy). The total EEG power (0.25 - 16 Hz range) was divided into 5 variable frequency bands: δ 0.25-3 Hz, θ 3-6; α₁ 6-9; α₂ 9-12 and β 12-16 Hz.

Qualitative and quantitative analysis on the electrocortical activity EEG recording was subsequently carried out.

### 3) Choline specific transport system (HUCT) at the pre-synaptic level

The animals were intracerebroventricularly (ICV) administered with *Hemicholinium-3* (Hch3), a potent and selective choline uptake blocker, in order to ascertain one of the suggested action mechanisms.

### 4) Lesion of Meynert nucleus basalis (NBM)

Male Wistar rats (200-250 g), anaesthetised with chloral hydrate (400 mg/kg), were subjected to unilateral lesion of Meynert nucleus basalis (NBM), by intracerebral infusion of α-amino-3-hydroxy-4-isoxazolepropionic acid (AMPA) (1.3 µg/0.5 µl). The animals were divided into four groups, the first two were subjected to NBM lesion; the third was a sham-operated group, and the last was the control group. Two weeks after the lesion, the animals were implanted under general anesthesia, with cortical electrodes (n = 4) onto the fronto-parietal region, so as to make it possible, 24 hours later, to record the EEG activity of freely moving animals. EEG activity was continuously monitored and recorded for 60 min by a computerised EEG Berg-Fourier apparatus. Spontaneous activity of the EEG spectrum total power (0.25 - 16 Hz), as well as that of preselected frequency bands, was recorded for times of 5 min and quantitized (µV).

### 5) Morphological study

In order to quantitize AMPA-induced neuronal loss in NBM, coronal serial sections of rat brain were prepared, then stained with toluidine blue to verify the injection site and treated, by immunohistochemical procedure, with 192LR-NGF to quantitize the neuronal loss. Said sections were analysed by a computerized system for image analysis (Axiophoy Zeiss and Vidas Kintron). Statistical analysis of the data from the morphological study was performed by ANOVA followed, if significant, by the *post-hoc* Tuckey-Kramer test.

### 6) Used compounds

The animals were divided in 4 groups, the first group acted as the control, the second received cytidine-5'-diphosphocholine (CDP-choline), the third and fourth received PC-1 and PC-2, respectively (pivaloylcholine iodide and pivaloylcholine trimethylacetate, respectively). The control group received an equal volume of saline solution by the same procedure.

### 7) Statistical analysis

The data from EcoG recording, carried out for epochs of 5 min, were divided in a 30 min control period and, after administration of the compounds, in epochs of 15 min each. Statistical analysis was performed by analysis of variance (ANOVA) for multiple measurements followed, if significant, by the *post-hoc* Tuckey-Kramer test. A comparison between groups was performed to exclude any individual variability.

### RESULTS

Intraperitoneal administration of PC-1 (5 mg i.p.) in rats produced, 15 min later, a behavioural response of *alert,* EEG desynchronization and significant decrease (P<0.05) in the EEG spectrum total power lasting 15 min (Fig. 1C and 3; Tab. 1 and 2); the fall in the total EEG power concerned the lowest frequency bands (0.25-3 and 3-6 Hz). In the rat, administration of PC-2 (5 mg i.p.) induced , within 15 min, *alert* response, EEG desynchronization and significant decrease (P<0.01) in the ECoG total power as well as in lower frequency bands (0.25-3 Hz) lasting about 30 min, (Fig 1D and 3; Tab. 1 and 2). It should be noted that, 60 min after administration of PC-2, EEG showed rapid frequency activity (about 16 Hz) and significant increase (P<0.01) in EcoG total power (Fig. ID). Subsequent quantitative analysis of EEG spectrum evidenced that said change was due to a significant increase (P<0.05) of β rhythm (12-16 Hz). Administration of CDP-choline (5 mg i.p.), used as the control drug, induced, 60 min after administration, EEG desynchronization and significant decrease (P<0.05) in EcoG spectrum total power (Fig. 1B and 3; Tab. 1 and 2) as well as in 0.25-3 and 3-6 Hz frequency bands. Administration of atropine, 15 min before administration of CDP-choline, PC-1 and PC-2, was able to antagonize the effects evoked by the administration of all said precursors of acetylcholine synthesis (Fig. 2). As a further control, administration of vehicle in a group of animals, by the same procedures and in the same volume, induced no changes in behaviour and EEG (Fig. 1A, Tab. 1 and 2).

Furthermore, ANOVA analysis evidenced a highly significant result, i.e. the drug x time interaction: F=4.77 (4, 112) P<0.001 and the drug x drug interaction: F=16.4 (28, 112) P<0.0001.

The EEG patterns and behavioral effects observed in the rat after administration of the choline derivatives and of the vehicle is reported in Tab. 3. Such effects can be ascribed to the changes of EEG spectrum total power. An increase in EEG total power is related with the presence of slow frequency waves (synchronisation), whereas an increase thereof is related with the presence of rapid frequency waves (desynchronisation).

ICV administration of Hch3 (1 µg - 0.1 ng) induced in the animal behavioural sedation and sleep, EEG synchronization and increase in EEG total power (0.25 ― 16 Hz).

Intraperitoneal administration of PC-1 in the animal at the active dose, after Hch3 pretreatment, induced neither *alert response* not EEG desynchronization, which effects were converesely observed following administration of PC-1 alone (Fig. 4b).

In lesioned animals (n=16), with about 45% neuronal loss at the NBM level (Fig. 5), EEG synchronization accompanied with significant increase (P < 0.01) in total power (0.25-16 Hz), (170.4±25,8) of EEG spectrum (µV), as well as in higher frequency bands (6-9, 9-12 and 12-16 Hz) (Fig. 7; Tab. 4), was observed. EEG activity of half the NBM lesioned animals was further recorded. After recording the baseline EEG activity, this group of animals was intraperitoneally administered with a PC-1 single dose.

Intraperitoneal administration of PC-1 (5 mg i.p.) induced, within 15 min, behavioural response of *alert,* EEG desynchronization and significant decrease (P<0.05) in ECoG spectrum total power lasting about 15 min (150 ± 19.3; 96 ± 20.3). Subsequent quantitative analysis of EEG spectrum revealed that said change was due to a significant decrease (P<0.05) in δ rhythm (0.25-3 Hz), (Fig. 6 and 7; Tab. 4).

Animals used as controls (n=12), (101±6.8) or sham-operated (n=12), (106±6.7), showed neither qualitative nor quantitative EEG changes of behaviour and cerebral electrical activity, (Fig. 7 and Tab. 4).

### Example 3 -Synergistic effect

Administration of tacrine (THA, 5 mg/Kg i.p.) 30 min before intraperitoneal injection of PC-1 and PC-2 (15 µmol i.p.) (n = 7 animals per each test) increase the EEG effects of both derivatives (carrier) producing long-lasting EEG desynchronization and significant decrease in EEG spectrum total power (Tab. 2) as well as of low frequency bands (0.25-3 and 3-6 Hz). The EEG changes were accompanied by behavioural *alert* response. Results reported in Table 5 show the synergistic effect of the combination of tacrine with the choline derivatives of the present invention.

### TABLES

**Table 1 Quantitative analysis of the different EEG frequencies in rats after administration of the various choline derivatives.**

| **Band (Hz)** | **Control** | **CDP-choline** | **PC-1** | **PC-2** |
|---|---|---|---|---|
| 0.25-3 | 6.4±0.3 | 3.8±1.3(*) | 4.3±0.7(*) | 2.4±1.3(**) |
| 3-6 | 28.5±0.9 | 19.2±1.5(*) | 18±0.6(*) | 21.3±0.9 |
| 6-9 | 27.6±1.0 | 31.5±0.9 | 32.5±1.3 | 22.3±1.3 |
| 9-12 | 14±0.6 | 18.5±1.7 | 19.3±0.9 | 13.5±0.7 |
| 12-16 | 15.3±0.6 | 20.5±1.3 | 13±1.0 | 22.5±1.6(*) |

| | | | | |
|---|---|---|---|---|
| (*)= P<0.05; (**)= P<0.01 vs control. Values are expressed as % ± SEM | | | | |

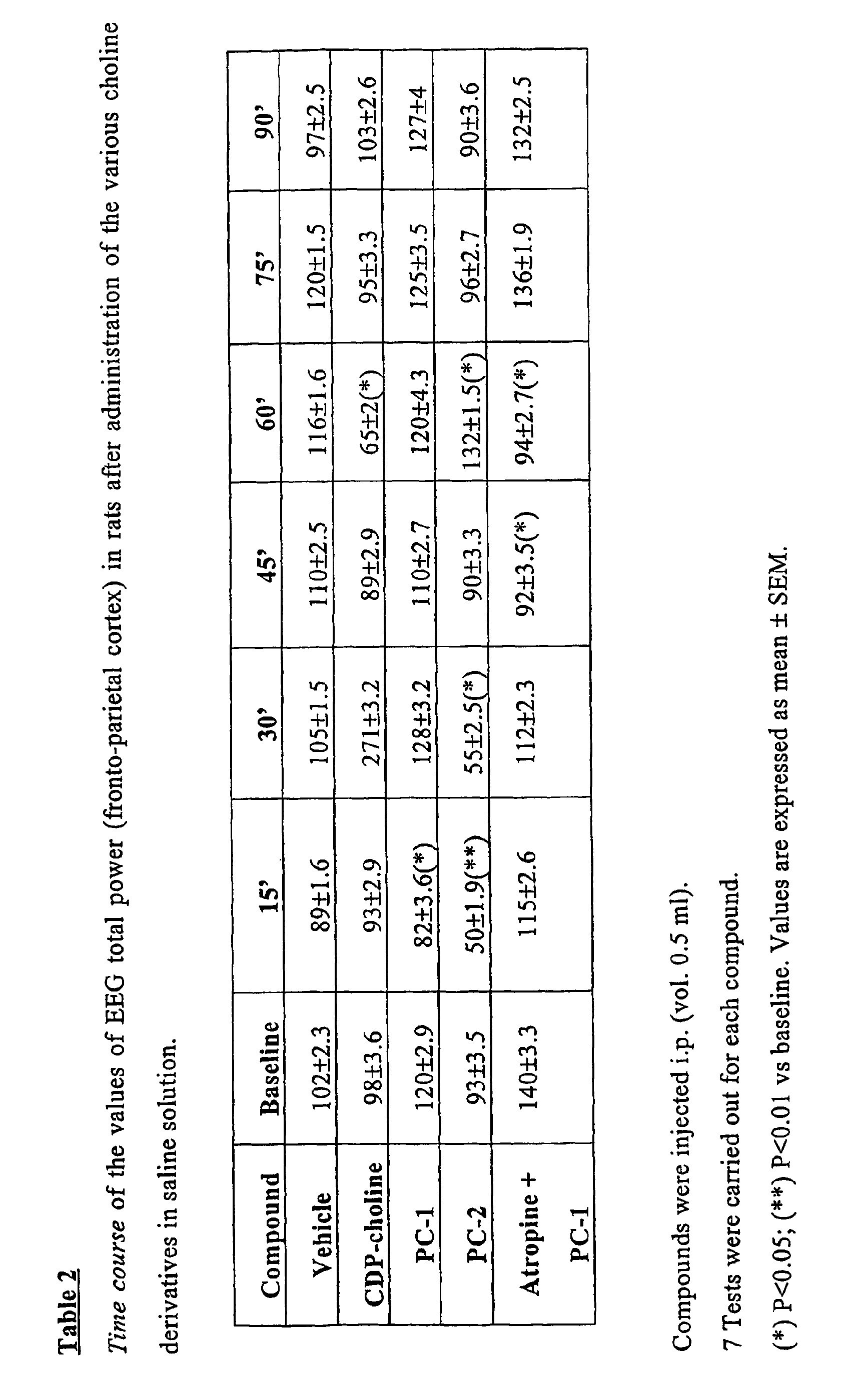

### Table 3

Behavioural and EEG effects observed in rats after administration of the vehicle (same volume of saline solution administered with the same procedure) and of various choline derivatives in different doses.

The EEG effects are ascribable to the changes of the total power (µV) of the EEG spectrum. An increase in EEG total power is related with a prevailing presence of slow frequency waves (synchronisation), whereas its decrease is related with the presence of rapid frequency waves (desynchronisation). The elements indicated as spikes are an index of suffering and/or neuronal damage; from the behavioural point of view they are, in fact, related with convulsive manifestations.

| | | EEG Activity | Behaviour |
|---|---|---|---|
| **Vehicle** | | No effects | No effects |

| **CDP-choline** | | | |
|---|---|---|---|
| | **50 mg** | No effects | Sedation |
| | **25 mg** | Increase | Sedation |
| | **5 mg** | Decrease | Alert response |
| | **1 mg** | No effects | No effects |

| **PC-1** | | | |
|---|---|---|---|
| | **50 mg** | Peaks | Convulsions and death (50%) |
| | **5 mg** | Decrease | Alert response |
| | **1 mg** | Decrease | Alert response |

| **PC-2** | | | |
|---|---|---|---|
| | **50 mg** | Peaks | Convulsions and death (50%) |
| | **5 mg** | Decrease | Alert response |
| | **1 mg** | Not tested | Not tested |

### Table 4

Quantitative analysis of EEG spectrum total power in different groups of animals with and without NBM unilateral lesion. Comparison was carried out on the ipsilateral hemisphere to the lesion.

| **Band (Hz)** | **Control** | **Sham** | **Lesions** | **Lesions + PC-1** |
|---|---|---|---|---|
| 0.25-3 | 5.1 | 3.4 | 8.8 | 3.4 |
| 3-6 | 30.8 | 30.6 | 39.1 | 33.3 |
| 6-9 | 28.3 | 29.2 | 45.6* | 23.5 |
| 9-12 | 18.6 | 19.5 | 36.2* | 13.6 |
| 12-16 | 18.9 | 22.2 | 40.6** | 14.6 |

| | | | | |
|---|---|---|---|---|
| (*)=p<0.05; (**)=P<0.01 vs control. Values are expressed as %. | | | | |

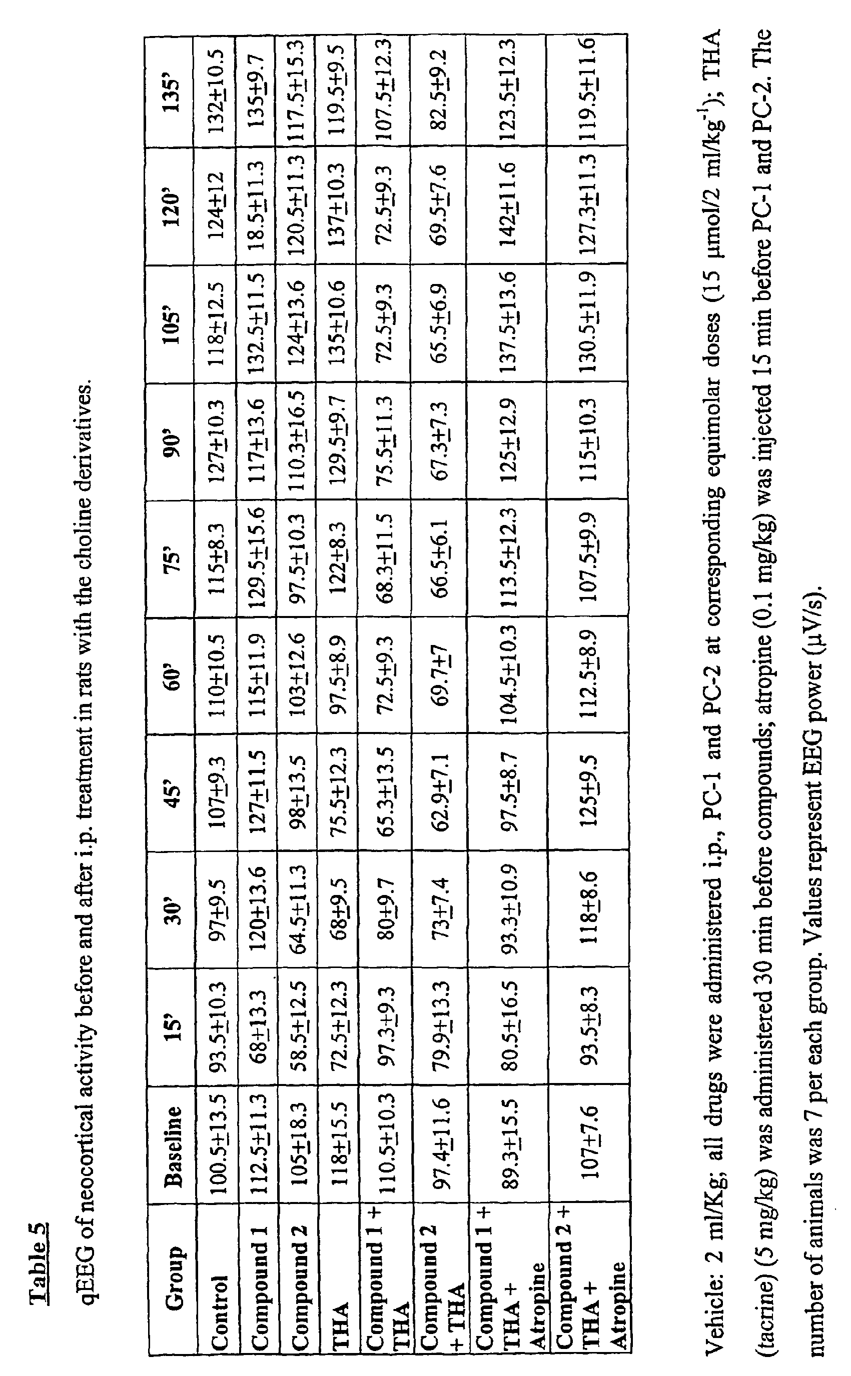

### REFERENCES

1. Arendt et al., 1984, Neurosci. Lett. 48: 81-85.
2. Casamenti F. et al., 1986, Brain Res. Bull. 16: 689-95.
3. Chiaramonti R. et al., 1997, Neuropsychobiology 36(3):153-158.
4. Cohen et al., 1985; 1990.
5. Deeker MA., 1987, Brain Res. Review 12: 424-438.
6. De the Morena E., 1991 Ann. N.Y. Acad. Sci. 640:233-235.
7. D'Onofrio F. et al., 1996, Acta Neurol Scand. 93(5):336-345.
8. Elmstahl S. et al., 1994, Dementia 5(2): 119-124.
9. Franco-Maside A. et al., 1994, Methods Find Exp Clin Pharmacol, 16(8): 597-607.
10. Funahashi S., 1983, Brain Res. 276: 267-276.
11. Greenwald BS. et al., 1985, Biol. Psychiatry 20(4): 367-374.
12. Helkala EL et al., 1996, Behav. Neurosi. 110(6): 1235-1243.
13. Lo Conte G. et al., 1982, Arc. Ital. Biol. 120: 176-188.
14. Longo VG., 1966; Pharmacol. Rev. 965-96.
15. Kuskowski MA. et al., 1993, Biol. Psychiatry 33(8-9): 659-662.
16. Lopez OL et al., 1997, Neurology 48(6): 1521-1525.
17. Mesulam MM. et al., 1983, J. Comp. Neuronal 214: 170-197.
18. Miller F. et al., 1940. Neurophysiol, 3: 131-138.
19. Moruzzi, G., 1972, Ergeb. Physiol. Biol. Chem. Exp. Pharmacol. 64: 1-165.
20. Ono Y. et al., 1992, Jpn J Psychiatry Neurol. 46(3): 659-664.
21. Perry EK. et al., 1978, J. Neurochem. 42: 1402-1410.
22. Rinne et al., 1987, J. Neurol. Sci. 79: 67-76.
23. Schreiter-Gasser U. et al., 1993, Electroencephalogr. Clin. Neurophysiol. 86(1): 15-22.
24. Secades JJ. et al., 1995, Methods Find Exp Clin Pharmacol. 17 Suppl B 2-54.
25. Soininen H. et al., 1991, Acta Neurol. Scand. 83(2): 133-136.
26. Stam CJ et al., 1996, Clin Electroencephalog 27(2):69-77.
27. Stem WC. And Pugh WW., 1984, Society for Neuroscience Abstract 10.9.
28. Stewart DJ. et al., 1984, Brain Res. 322:219-232.
29. Whitehouse PJ. et al., 1982, Science 215: 1237-1239.
30. Whitehouse PJ. et al., 1981, Ann. Neurol. 10: 122-126.
31. Gatti G. et. Al., 1992, Int. I. Clin. Pharmacol. Ther. Toxical. 30: 331-335.
32. Ceda G.P. et al., 1992, Horm. Metab. Res. 24: 119-121.
33. Parnetti L. et al., 1993, Drugs and Againg 3: 159-164.
34. Petkov V. D. et al., 1993, Arzneimittelforschung 43: 822-828.
35. Cacabelos R. et al., 1993, Ann. N.Y. Acad. Sci. 695: 321-323.

## Claims

1. Compounds of formula (I):
(CH₃)₃C-COO-CH₂CH₂-N⁺(CH₃)₃ X⁻
wherein X⁻ is iodide or the pivalic acid anion, for use in the treatment of diseases related with central cholinergic deficit.

2. Compounds as claimed in claim 1, for the treatment of Alzheimer's disease or of Alzheimer-type diseases.

3. A combination of a compound of formula (I) with tacrine for the simultaneous, separated or sequential use in the treatment of diseases related with central cholinergic deficit.

4. A combination as claimed in claim 4, for the treatment of Alzheimer's disease or of Alzheimer-type diseases.

5. A pharmaceutical composition containing one or more compounds of formula (I), optionally in combination with tacrine, together with pharmaceutically acceptable vehicles and excipients.

## Patentansprüche

1. Verbindungen der Formel (I):
(CH₃)₃C-COO-CH₂CH₂-N⁺(CH₃)₃ X⁻,
worin X⁻ Jodid oder ein Pivalinsäureanion ist, zur Verwendung in der Behandlung von Erkrankungen, die im Zusammenhang mit einem zentralcholinergen Defizit stehen.

2. Verbindungen nach Anspruch 1, für die Behandlung der Alzheimer-Erkrankung oder von Erkrankungen des Alzheimertyps.

3. Eine Kombination einer Verbindung der Formel (1) mit Tacrin für die gleichzeitige, getrennte oder sequentielle Anwendung in der Behandlung von Erkrankungen, die im Zusammenhang mit einem zentralcholinergen Defizit stehen.

4. Eine Kombination nach Anspruch 4 für die Behandlung der Alzheimer-Erkrankung oder von Erkrankungen des Alzheimertyps.

5. Eine pharmazeutische Zusammensetzung enthaltend eine oder mehrere der Verbindungen der Formel (1), wahlweise in Kombination mit Tacrin, zusammen mit pharmazeutisch verträglichen Vehikeln und Exzipienten.

## Revendications

1. Composés répondant à la formule (I) :
(CH₃)₃C-COO-CH₂CH₂-N⁺(CH₃)₃ X⁻
dans laquelle X⁻ représente un anion iodure ou l'anion de l'acide pivalique, à utiliser dans le traitement de maladies associées à un déficit cholinergique central.

2. Composés selon la revendication 1, pour le traitement de la maladie d'Alzheimer ou de maladies de type Alzheimer.

3. Combinaison d'un composé répondant à la formule (I) avec de la tacrine pour l'utilisation simultanée, séparée, ou séquentielle, dans le traitement de maladies associées à un déficit cholinergique central

4. Combinaison selon la revendication 4, pour le traitement de la maladie d'Alzheimer ou de maladies de type Alzheimer.

5. Composition pharmaceutique contenant un ou plusieurs composés répondant à la formule (1), éventuellement en combinaison avec de la tacrine, ensemble avec des véhicules et des excipients pharmaceutiquement acceptables.
